# EUROPEAN PATENT APPLICATION

(11) **EP 2 559 418 A1**
(43) Date of publication of application: **20.02.2013**
(21) Application number: 11180352.4
(22) Date of filing: 07.09.2011
(51) Int. Cl.: A61K 8/04, A61K 8/44, A61K 8/81, A61Q 9/04

(54) **Fragrant depilatory article**

(30) Priority: 17.08.2011 US 524364 P
(71) Applicant: The Procter & Gamble Company, Cincinnati, OH 45202 (US)
(72) Inventor: Smith, Paul James, Whitton, Middlesex TW2 6EB (GB); Plos, Julien Claude, London, W4 4JB (GB); Evangelista, Olindo, 66023 Francavilla Al Mare (IT); Sagel, Paul Albert, Maineville, OH Ohio 45039 (US); Passi, Rajeev Kumar, West Chester, OH Ohio 45069 (US); Mitra, Shekhar, Cincinnati, OH Ohio 45243 (US); Broyles, Norman Scott, Hamilton, OH Ohio 45011 (US)
(74) Representative: Wilding, Richard Alan

(57) **Abstract**

A depilatory article comprising a substrate and a depilatory composition disposed on said substrate wherein the depilatory composition comprises solid entrapping particles, said particles comprising a polymer and a volatile material or mixture of volatile materials.

## Description

### FIELD OF THE INVENTION

The present invention relates to depilatory articles comprising a chemically active depilatory composition disposed on a substrate.

### BACKGROUND OF THE INVENTION

Depilatory compositions used to remove unwanted hair by chemical activity are known. Such compositions may comprise reducing agents to degrade keratin in the hair and thus weaken the hair strands. These compositions may take the form of creams, lotions and the like which may be applied to the unwanted hair in a variety of ways, such as with a spatula. The spatula or another suitable implement is then used to scrape off the weakened hair strands and complete the depilation process. This can be a messy and awkward procedure for the user of the depilatory cream or lotion and provides no means of occluding the depilatory composition to prevent it from drying out. By disposing the depilatory composition on a substrate one may overcome or mitigate such disadvantages. Substrate-based depilatory products are known from JP6192056A, US2006002878, JP6135826A, JP11012123A and JP62230711A. JP63073910A in particular considers a high water content composition disposed on a substrate.

While addressing some of the usage problems of creams and lotions by removing the need for an application implement, known substrate-based depilatory compositions do not address the problem of reducing the malodor that arises from the typically sulphur-containing keratin reducing agents that provide activity to the depilatory composition. A possible solution may be to incorporate a fragrance into the depilatory composition to mask said malodor. However, fragrances and perfumes can result in instability when used in combination with a substrate in the form of a depilatory article as they may be drawn to the surface of the substrate and therefore separate from the depilatory composition. This can affect the adherence of the depilatory composition to the substrate as well as the effectiveness of the perfume. For example, perfume may be largely trapped beneath the depilatory composition and unable to be detected, or may appear at the surface, causing an immediate burst of aroma, but one that then fades while the malodor resulting from the depilatory process is worsening. There exists a need, therefore, for a substrate-based depilatory article in which perfumes may be stably incorporated.

### SUMMARY OF THE INVENTION

According to a first aspect of the invention, the Applicants have surprisingly found that a depilatory article comprising a substrate and a depilatory composition disposed on said substrate wherein the depilatory composition comprises solid entrapping particles, said particles comprising a polymer and a volatile material or mixture of volatile materials meets the aforementioned need by protecting the perfume from interacting with the depilatory composition in which it is incorporated.

According to a second aspect of the invention, a cosmetic method of removing hair from the skin is provided, comprising the steps of applying a depilatory article according to the first aspect of the invention to a surface of the body, preferably the human body, leaving said depilatory article in contact with the skin for a period of at least 1 minute, preferably 2 to 10 minutes, more preferably 2 to 8 minutes, removing said depilatory article from the surface of the skin, and preferably rubbing, scraping, rinsing or wiping the surface of the skin in the area to which the depilatory article was applied.

According to a third aspect of the invention, a depilatory kit is also provided, comprising a depilatory article according to the first aspect of the invention, optionally at least one of a pre-treatment skin care composition, a post-treatment skin care composition and/or a tool to assist removal of hair and/or depilatory composition after use, and packaging for said depilatory kit.

### BRIEF DESCRIPTION OF THE DRAWINGS

Fig.1. is a plan view of a depilatory article of the present invention.
Fig.2. is a side view of a depilatory article of the present invention.
Fig.3. is a side view of a depilatory article of the present invention applied to keratinous tissue.

### DETAILED DESCRIPTION OF THE INVENTION

As used herein, the term "buffering base" refers to a base capable of opposing pH changes by means of chemical or physical (solubility) processes and thereby limiting the pH to less than or equal to 13.

As used herein, the term "water impermeable" includes materials or objects through which water in its liquid state does not pass.

As used herein, the term "water soluble" includes materials which have a solubility of at least 0.01 g/dm³, preferably at least 0.1 g/dm³, more preferably at least 0.5 g/dm³, even more preferably at least 0.8 g/dm³ and even more preferably still at least 1 g/dm³.

As used herein, the term "solid" includes the state of matter as distinct from liquid or gaseous, and especially describes a uniform structure in respect of the state of matter, i.e. "solid throughout" as distinct from either a hollow structure, or one having a liquid or vapour core, but including a solid matrix comprising a liquid or gas, for example a liquid absorbed into said matrix.

As used herein, the term "volatile" includes materials with a boiling point at standard pressure below 200°C, preferably below 150°C, more preferably below 100°C, even more preferably below 75°C and even more preferably still below 50°C.

As used herein, all percentages are by weight of the depilatory composition, unless otherwise specified.

Depilatory articles of the present invention comprise a depilatory composition in contact with a surface of the substrate, preferably forming a coated region of the substrate. The depilatory composition may be disposed on one surface of the substrate, that surface being a depilatory surface of the substrate. The depilatory composition should be suitable for being placed in contact with a user's skin (and unwanted hair). Advantageously, the depilatory composition is an aqueous gel, and/or the concentration of water in the depilatory composition is equal to or greater than 40%, preferably from 50% to 95%, more preferably from 60% to 90% and even more preferably from 70% to 90%, by weight of the depilatory composition. This high water level helps to improve the overall skin mildness of the depilatory composition by being dilute, and to keep the system more robust to pH changes, which may result in skin irritation.

The depilatory composition comprises solid entrapping particles, preferably from 0.01% to 10%, more preferably from 0.1% to 5%, even more preferably from 0.2% to 3% even more preferably still from 0.5% to 2% and yet more preferably 0.7% to 1.5% solid entrapping particles by weight of the composition.

The solid entrapping particles comprise a polymer and a volatile material or mixture of volatile materials. Preferably, the solid entrapping particles comprise from 10% to 98%, more preferably from 15% to 80%, even more preferably from 18% to 50%, and even more preferably still from 20% to 40%, volatile material by weight of particle. The solid entrapping particles thus may allow the incorporation of a high proportion of volatile materials.

The solid entrapping particles can be of different shapes depending on the process used to produce them and the depilatory composition into which they are incorporated. For example, when dispersed in an aqueous solution or gel, solid particles generally have a substantially spherical shape. When dispersed in an emulsion, solid particles typically have a more irregular shape, e.g. the shape of an irregular star. Without wishing to be bound by theory, it is believed that the shape of the solid entrapping particles is dependent, at least partially, on the difference in surface energy between the depilatory composition and the solid entrapping particles. When the difference is maximal, the solid entrapping particles are spherical. As the difference of surface energy diminishes, the solid entrapping particles become more irregular in shape. Whatever the shape of the solid particles, the inventors have found that an improved sustained, uniform release of volatile materials without significantly compromising depilatory effectiveness or greatly increasing skin irritation can be achieved when solid entrapping particles have an average particle size from 5 to 200 micrometers, preferably from 30 to 100 micrometers, more preferably from 30 to 50 micrometers.

The average particle size can be determined by using any appropriate instrument known by the skilled person. For example, the average particle size may be measured with a Lasentec D600L instrument, supplied by Mettler Toledo, using a technique known as Focused Beam Reflectance Measurement (FBRM) which provides continuous in-process and real-time measurement of the rate and degree of change of the particle size and particle count. In this technique, a focused laser beam rotates at high speed and propagates into the particle suspension to be monitored. When the focused laser beam crosses a particle in front of the probe, a signal is backscattered into the probe. The length of the scanned particle (or size) is determined by the electronics of the system and transferred into a diameter length distribution histogram. The diameter length distribution provides particle count and particle size information from which an average particle size may be determined.

The polymer of the solid entrapping particles may be of any suitable type, for example any thermosoftening polymer such as a polyolefin as well as thermosoftening polymers comprising a heteroatom, such as PVA. In a particularly preferred embodiment, the solid entrapping particles comprise a polymer comprising ethylene monomer units.

The polymer preferably has a low melting point. As used herein, the term "low melting point" means a melting point below 80°C, preferably from 45°C to 80°C and more preferably from 45°C to 55°C. The melting point can be measured by conventional methods. Polymers having a low melting-point are preferred so that the polymeric composition can be formulated at a low application temperature, i.e. at a temperature below 100°C, and then these polymeric compositions can be incorporated into the cosmetic composition also at a low application temperature. The use of a low application temperature is desirable to prevent the evaporation of a significant amount of volatile materials and also to prevent the degradation and/or the inactivation of any cosmetic active components when incorporating the polymeric composition into the depilatory composition.

In a preferred embodiment, the polymer is a copolymer obtainable by copolymerization between monomers of ethylene and at least one class of co-monomer comprising at least one heteroatom. In such an embodiment, the copolymer may comprise, therefore, ethylene monomers and co-monomers comprising at least one heteroatom.

The term "co-monomer comprising at least one heteroatom" includes monomers comprising at least a polar C-X linkage with X being neither a carbon atom nor a hydrogen atom. X can be, for example, a nitrogen, sulphur, fluorine, chlorine or oxygen atom. Preferably, the co-monomer comprises a carbonyl group, more preferably the co-monomer comprises an ester group, more preferably selected from a vinyl ester, an acrylic ester, a methacrylic ester and mixtures thereof. Still more preferably the co-monomer is a vinyl ester. A particularly preferred co-monomer is a vinyl acetate ester and the preferred copolymer is, therefore, an ethylene-vinyl acetate copolymer (or EVA copolymer). Without wishing to be bound by theory, it is believed that EVA copolymers are a more effective copolymer in improving the sustained release of volatile materials.

The co-monomer preferably represents from 10% to 90%, more preferably from 14% to 80%, still more preferably from 18% to 70% by weight of the total copolymer.

The copolymer may further comprise other co-monomers not being a co-monomer comprising at least one heteroatom. For example, the copolymer may further comprise crosslinking co-monomers.

Copolymer raw materials are commercially available. For example, suitable ethylene-vinyl acetate copolymer raw materials are sold under the trade names: Elvax™ by Dupont, Evathane™ by Atofina, Escorene™ by Exxon, Levamelt™ and Levamelt™ by Bayer. Particularly preferred are Elvax™ 250 having a melting point of 70°C and Elvax™ 40W having a melting point of 47°C. A suitable ethylene-acrylic ester copolymer raw material is sold under the trade name Lotryl™ by Atofina.

The solid entrapping particles comprise at least one volatile material or mixture of volatile materials. The volatile materials can be selected from flavors, deodorants, insecticides, pheromones, aromas, repelling agents, perfumes or a mixture thereof. Preferably, the volatile material is, or comprises, a perfume. As used herein, the term "perfume" means any odiferous material comprising one or more volatile components, i.e. components having a vapor pressure less than the atmospheric pressure at room temperature. Depending on their volatility, these components tend to evaporate more or less quickly. The higher the volatility is, the quicker the evaporation. Components evaporating the most rapidly are usually classified as top notes and components evaporating the least rapidly as base notes, intermediary components being classified as middle notes.

More preferably, perfume comprises more than one component having a vapor pressure less than the atmospheric pressure at room temperature. When said perfume comprises more than one of these components, the components usually have different volatility. When these components are merely incorporated into a composition, these components tend to evaporate at different speed depending on their volatility upon use. Due to this differential evaporation, the consumer may perceive different scents over time. In contrast, when a composition comprises a perfume comprised within the solid entrapping particles according to the invention, the release of the perfume would be more uniform over time. Without wishing to be bound by theory, it is believed that the release of these volatile components over the time is not merely driven by their volatility. In contrast, it is believed that their release is dependent upon several factors - including their volatility, their molecular weight, their chemical interactions with the other components of the solid entrapping particles and the total proportion of volatile materials in the solid entrapping particles in combination with the specific average particle size of the solid entrapping particles - driving therefore a sustained, uniform release over the time. Perfume can be in a liquid or solid form at room temperature and be obtained from a natural source, a synthetic source or a mixture thereof. Advantageously, the perfume appropriately masks the particular malodor arising from depilatory compositions, and/or remains stable in them irrespective of the entrapment offered by the present invention.

Preferably, the perfume comprises at least one component being selected from an aldehyde, a ketone, an ester, an alcohol or a terpene. These components may be selected depending on the scent desired. These components are well-known by the skilled person. For illustration, perfume materials are described more fully in S. Artander, Perfume Flavors and chemicals, Vols I and II, Montclair, N.J. and the Merck Index, 8th Edition, Merck & Co., Rahway N.J.

In an advantageous embodiment, the particles comprise a plasticizer. The plasticizer may comprise at least one heteroatom. Advantageously, the plasticizer is compatible with the polymer, especially the copolymer obtainable by copolymerization between monomers of ethylene and at least one class of co-monomers comprising at least one heteroatom.

As used herein, the term "compatible" means that the water solubility of the copolymer matches the water solubility of the plasticizer at ambient temperature (i.e. at 25°C) to within 1 g/dm³. When a copolymer and a compatible plasticizer are mixed, a homogeneous mixture is obtained and the compatible plasticizer exhibits a plasticizing effect as it lowers the glass transition temperature of the polymer. The compatibility of the polymer and the plasticizer may be determined by using any conventional method known by the skilled person, e.g. water/octanol partition coefficient.

The term "plasticizer comprising at least one heteroatom" includes plasticizers comprising at least a polar C-X linkage wherein X is neither a hydrogen atom nor a carbon atom. X can be, for example, a nitrogen, sulphur, fluorine, chlorine or oxygen atom. Preferably, the plasticizer comprises a carbonyl group and more preferably an ester group. When the plasticizer comprises an ester group, the plasticizer is preferably selected from an adipic acid ester, a benzoic acid ester, a citric acid ester, a phosphoric acid ester, a phthalic acid ester, a rosin ester, a sebacic acid ester, a sucrose ester, a tartaric acid ester or mixture thereof. More preferably, the plasticizer is selected from an adipic acid ester, a citric acid ester, a rosin ester, a sebacic acid ester or mixture thereof. Still more preferably, the plasticizer is selected from citric esters and/or sebacic esters as both citric esters and/or sebacic esters exhibit a satisfactory compatibility with the copolymer according to some embodiments of the invention and are safe for skin application.

Plasticizer raw materials are commercially available. For example, suitable esters of hydrogenated rosin are sold under the trade name Foralyn 5020-F™ by Eastman. Suitable esters of citric acid are sold under the trade name Citrofol BII™ (acetyl tributyl citrate) by Jungbunzlauer; Citrofol AH II™ (acetyl 2-ethylhexyl citrate) by Jungbunzlauer, and; Morflex MSC™ (monosteryl citrate) by Morflex. Suitable esters of sebacic acid are sold under the trade name Morflex DBS™ (dibutyl sebacate) by Morflex.

The solid entrapping particles may preferably, for example, comprise, by weight of particle, from 5% to 75% polymer, from 10% to 50% plasticizer and from 10 to 85% volatile material.

In a particularly preferred embodiment, the solid entrapping particles comprise from 5% to 75%, more preferably from 10% to 50%, copolymer obtainable by copolymerization between monomers of ethylene and at least one class of co-monomers comprising at least one heteroatom, from 10% to 50%, more preferably from 15 to 40%, plasticizer comprising at least one heteroatom and from 10 to 95%, more preferably from 20 to 90%, still more preferably from 30% to 85%, and most preferably from 40% to 80%, volatile material by weight of particle in order to obtain a good balance between the physical characteristics of the particles and the perfume strength and release.

Depilatory articles of the present invention comprise a substrate to facilitate application of the depilatory composition to keratinous tissue and prevent a messy usage experience.

Advantageously, the substrate possesses a rigidity in the range of from 5.00 g/cm to 0.05 g/cm, preferably from 3.00 g/cm to 0.05 g/cm and more preferably from 1.80 g/cm to 0.08 g/cm, even more preferably from 0.80 g/cm to 0.10 g/cm and even more preferably still from 0.60 g/cm to 0.20 g/cm. This rigidity of the substrate promotes desirable handleability and conformability attributes of the depilatory article. In particular, the article collapsing under gravity or folding is avoided, which is especially undesirable if different areas of the depilatory composition are able to readily come into contact with each other, while maintaining the capability for the depilatory article to conform to the surface to which it is applied without folding or crinkling, in order to further improve depilatory efficiency. Accordingly, the substrate is readily conformable to the skin and unwanted hair without permanently deforming during use, as this may also result in problems for the user during application. In a preferred embodiment, the rigidity is substantially constant and does not change during the lifetime of a product.

Rigidity can be readily measured using the American Standard Test Method (ASTM) D2923-06, method B (i.e. using a powder to reduce the effect of static electricity), on a Handle-O-Meter, model #211-300, available from Thwing-Albert Instrument Co. of Philadelphia, Pa. The rigidity is read directly from the meter and expressed as grams per centimetre of sample width. Samples were prepared as 10.16 cm (4 inch) by 10.16 cm (4 inch) test specimens with edges parallel to the machine direction and transverse direction for substrates with directionality. Three rigidity measurements were determined on the same side of fresh test specimens orientated in the same substrate direction. A further three rigidity measurements were taken on the same side of fresh test specimens oriented at 90° to the first orientation. These six measurements were repeated on the opposite side to the first six measurements, on fresh test samples. The 12 rigidity measurements were then averaged and reported to 0.01 g/cm.

The rigidity of a substrate is a function of substrate thickness and inherent modulus of elasticity. Different materials have different moduli of elasticity. Based upon the material or materials that the substrate comprises, a substrate thickness should be selected that enables the desired rigidity of the substrate to be achieved.

The substrate may be water permeable or water impermeable. The substrate may comprise any suitable material such as fibrous materials, papers, fabrics, non-wovens, plastics, amorphous solids, crystalline solids, foils, rubbers, latex, thermoplastic elastomers, cellular foams (open and closed cell), composites, laminates and mixtures thereof. Preferably, the substrate is water impermeable. Using a water impermeable substrate prevents water loss from the depilatory composition while the depilatory composition is in contact with the keratinous tissue and thus prevents the depilatory composition from drying out. Water loss from the depilatory composition lowers the water concentration, thus increasing the concentration of active ingredients and bases present. This could result in irritation to the skin, which applicants wish to avoid, and may also affect the stability of the composition.

The substrate preferably comprises at least one water impermeable material and is compatible with depilatory compositions. Examples of useful water impermeable materials include but are not limited to polypropylene (PP); polyethylene (PE, including HDPE and LLDPE); polyethylene terephthalate (PET); polyvinylchloride (PVC); polyamide (PA); polycarbonate; polyurethane; cellulose acetate; polychloropene; polysulfone; polytetrafluoroethylene (PTFE); polyvinyl acetate (PVA); polystyrene; polyphenylene oxide (PPO); acrylonitrile butadiene styrene (ABS); acrylic; acrylonitrile styrene acrylate (ASA); ethylene vinyl alcohol (EVA); natural rubber, latex, nylon, nitrile, silicone and thermo plastic elastomers (TPE). The substrate may comprise a single polymer or mixtures of polymers or copolymers. Preferably the substrate comprises a plastic sheet, more preferably a polyolefin, even more preferably a polyethylene and even more preferably still high density polyethylene.

In an advantageous embodiment, the depilatory composition is disposed upon the water impermeable material, preferably plastic sheet, more preferably polyolefin, even more preferably polyethylene and even more preferably still high density polyethylene. In this advantageous embodiment, there is preferably no layer of water permeable material between the depilatory composition and the water impermeable material. In a preferred embodiment, the water impermeable material forms a water impermeable layer.

The substrate preferably has a thickness of from 80 µm to 12 µm, more preferably from 50 µm to 15 µm, even more preferably from 40 µm to 16 µm, and even more preferably still from 30 µm to 17 µm.

The substrate may be a laminate comprising at least two materials, including non-wovens; paper; board; metal based substrates (eg aluminium foil); flocking or topical coatings (e.g. surfactants; printing); closed or open cell foams or substrates described herein above. In a preferred embodiment, at least one of the materials is water impermeable.

The substrate may be manufactured by any suitable method, including casting, injection moulding, co-injection moulding, over moulding, in-mold assembly, compression moulding, blow moulding, casting thermo or vacuum forming and where appropriate may be laminated by heat welding (which may further include the use of pressure, ultrasonic forces and radio or high frequencies), co-extrusion; adhesives, electro static adhesions (such as flocking by fibres) and topical surface applications.

Depilatory articles of the present invention may advantageously comprise a protective release layer removably attached to the depilatory composition, preferably on a surface of the depilatory composition substantially opposing that which is in contact with the substrate.

The protective release layer may comprise materials including polymer resins such as polyolefins e.g. polypropylene (including stratified biaxially oriented polypropylene (SBOPP)), polyethylene (including LDPE; LLDPE; HDPE; Metallocene) or polyethylene terephthalate. Alternative materials which may be used include polyvinylchloride, polyamide, acetyl, acrylonitrile butadiene styrene, acrylic, acrylonitrile styrene acrylate, ethylene vinyl alcohol, ethylene vinyl acetate, Nylon, Latex, natural or synthetic rubbers, polycarbonate, polystyrene, silicone or thermo plastic elastomer, thermo plastic vulcanate or copolymers of said materials. Where appropriate the protective relase layer may comprise one or more laminations, combinations of multiple layers and/or indications (which may include instructions and illustrations) relating to at least one aspect of the usage of the depilatory article. In an advantageous embodiment the protective release layer may comprise a coating of a non-stick material. Exemplary non-stick coatings include wax, silicone, fluoropolymers such as TEFLON®, and fluorosilicones. In a preferred embodiment, the protective release layer covers at least the entire aforementioned coated region of the substrate. In another preferred embodiment the protective release layer is water impermeable. In a further preferred embodiment, the protective release layer has a mean thickness of at least 85 microns, more preferably from 85 microns to 130 microns, even more preferably from 90 microns to 120 microns. In yet another preferred embodiment, the protective release layer extends beyond the coated region of the substrate to provide a removal tab.

The rheological properties of the depilatory composition may also lead to improved performance in use. In particular, the yield point describes the resistance of the depilatory composition to deformation under environmental stress. If the yield point is too high, then the depilatory composition may not deform sufficiently, with hair fibres unable to enter the depilatory composition effectively upon application, resulting in less desirable depilatory effectiveness. If the yield point is too low, however, then the depilatory composition may flow during storage, transport or use, and may also permit sedimentation of the solid entrapping particles. Further, the depilatory composition may not be cleanly removed from the skin upon removal of the depilatory article, thus requiring the inconvenience of additional wiping and risking irritation to the user. A poor yield point is especially undesirable when a protective release layer is used as the composition may break apart as the substrate and release layer to which it adheres are pulled apart. Accordingly, the depilatory composition preferably has a yield point from 10 Pa to 2000 Pa, more preferably from 30 Pa to 1200 Pa, even more preferably from 45 Pa to 500 Pa and even more preferably still from 60 Pa to 300 Pa, when measured via a stress controlled amplitude sweep at a frequency of 1 Hz and a temperature of 25°C. The yield point described is defined herein as the 5% decrease in magnitude of the elastic modulus G' linear viscoelastic plateau value as measured on a TA1000 Rheometer, available from TA Instruments of New Castle, Delaware, USA. The rheological properties of the depilatory composition may be altered by changing the concentration or identity of the thickening system and the water content of the depilatory composition.

In another preferred embodiment, the depilatory composition displays a high degree of shear thinning behaviour enabling the effective coating of target hairs during application and improve depilatory efficacy. Accordingly, at a low shear rate of 0.1 s⁻¹, the dynamic viscosity of the depilatory composition is preferably 1000 Pa.s to 10000 Pa.s measured at a temperature of 25°C, whereas at a high shear rate of 1000 s⁻¹, the dynamic viscosity of the depilatory composition is preferably 0.1 Pa.s to 1 Pa.s, measured at a temperature of 25°C.

Preferably, the depilatory composition is disposed upon the substrate in an amount per unit area of 0.300 g/cm² to 0.001 g/cm², more preferably from 0.015 g/cm² to 0.003 g/cm², even more preferably from 0.080 g/cm² to 0.005 g/cm² and even more preferably still from 0.05 g/cm² to 0.005 g/cm², wherein the unit area refers to the coated region of the substrate and not including any uncoated surface of the substrate. Within these preferred ranges, sufficient depilatory composition is provided to coat the hairs to improve depilatory activity, but little enough to discourage rupturing of the depilatory composition when any protective release layer is removed from the depilatory article, or the depilatory article is removed from the surface of the body. Further, and particularly in combination with specific proportions of solid entrapping particles in the depilatory composition as disclosed herein, the amount of depilatory composition upon the substrate provides advantageous levels of volatile material and rates of release. In respect of the above, the area used to calculate the amount of depilatory composition disposed upon the substrate is calculated ignoring any surface texturing or micro-structuring. Alternatively, the mean thickness of the depilatory composition is preferably from 0.01 mm to 3 mm, more preferably 0.1 mm to 1.5 mm, even more preferably from 0.05 mm to 0.8 mm, and even more preferably still from 0.05 mm to 0.5 mm.

A layer of depilatory composition can be applied to the substrate through any known technique of applying viscous fluids to substrates, including, for example, extrusion, casting (e.g., reverse roll, knife-over roll, slot die, Gravure roll), spraying, knife blade coating, and zone coating. Such techniques may be modified to alter the quantity of depilatory composition disposed on the substrate. For example, the speed at which the substrate travels through an extrusion process determines the quantity of depilatory composition disposed upon said substrate. The area of depilatory composition may cover the entire surface of the substrate of a portion thereof. Advantageously, the depilatory composition covers less than the entire surface of the substrate to facilitate handling. The substrate may comprise at least one region with two orthogonal dimensions each of a length greater than 1 cm, preferably greater than 1.5 cm and more preferably greater than 2 cm upon which no depilatory composition is disposed.

In a preferred embodiment, the depilatory composition comprises a keratin reducing agent to weaken and/or break strands of unwanted hair. Non-limiting examples of suitable keratin reducing agents include: sulphide salts such as Li₂S, Na₂S, K₂S, MgS, CaS, SrS or BaS, hydrogen sulphide salts such as NaSH or KSH, thioglycol, thioglycerol, thioglycolamide, thioglycolhydrazide, thioglycolic acid, thioglycolate salts (such as potassium thioglycolate, calcium thioglycolate, ammonium thioglycolate, diammonium dithioglycolate, glyceryl monothioglycolate, or monoethanolamine thioglycolate), thiosalicylic acid, thiomalic acid, ammonium thiolactate, monoethanolamine thiolactate, dithioerythritol, 2-mercaptopropionic acid, 1,3-dithiopropanol, glutathione, dithiothreitol, cysteine, homocysteine, N-acetyl-L-cysteine and cysteamine. Advantageously, the keratin reducing agent is present in an amount of from 0.3% to 20%, preferably from 0.8% to 15%, more preferably from 1% to 10% by weight of the composition.

Advantageously, the depilatory composition may comprise at least one thioglycolate salt or thioglycollic acid acting as a hair removal agent when the depilatory composition is applied to unwanted hair. Preferably, the depilatory composition comprises sodium, potassium, magnesium, calcium, beryllium, strontium, zinc, monoethanolamine, ammonium, tetralkylammonium, imidazolium, pyridinium, phosphonium or glyceryl thioglycolate salts, or mixtures thereof, which may include dianion forms of thioglycolate. More preferably, the depilatory composition comprises at least one of sodium, potassium, magnesium or calcium thioglycolate, or mixtures thereof. Even more preferably the depilatory composition comprises potassium or calcium thioglycolate, or mixtures thereof. In a preferred embodiment, the concentration of the conjugate acid of the thioglycolate salt, (which includes all species in the acid's deprotonation equilibrium system) is from 0.5% to 12.0%, more preferably from 0.8% to 8.0% and even more preferably from 1.0% to 6.0% by weight of the depilatory composition.

In a preferred embodiment, the depilatory composition comprises a monovalent cation, preferably a monovalent metal cation. Without wishing to be bound by theory, the applicants believe that the presence of monovalent metal cations increases the dissociation of thioglycolate salts. The monovalent cations such as those derived from monovalent cation containing salts are able to displace the cation of the thioglycolate salt and further enhance dissociation of said thioglycolate salt. This increases the amount of deprotonated thioglycolate formed from the thioglycolate salt and therefore increases the effectiveness of the depilatory composition. Sources of monovalent cations include potassium, sodium, lithium, ammonium, tetraalkyl ammonium and imidazolium salts, which may be a component of another ingredient, for example a thickening system or skin care active. Preferred sources of monovalent cations include potassium and sodium salts.

In order to further enhance the safety of the resulting product, it is advantageous to limit the amount of monovalent cations, preferably monovalent metal cations, to which the skin is exposed when the depilatory article is used, although a small quantity may improve the efficacy of the depilatory composition. Advantageously, the quantity of monovalent cations (or monovalent metal cations in the preferred embodiment above) per unit area of the aforementioned coated region is less than 5.10 × 10⁻⁴ mol/cm², preferably less than 3 × 10⁻⁴ mol/cm⁻², more preferably from 1 × 10⁻⁹ mol/cm² to 1.5 × 10⁻⁴ mol/cm², even more preferably from 2.50 × 10⁻⁸ mol/cm² to 6.65 × 10⁻⁵ mol/cm² and even more preferably still from 6 × 10⁻⁷ mol/cm² to 4.5 × 10⁻⁵ mol/cm². The selection of keratin reducing agent and optional ingredients including the base may be made considering the quantity of monovalent cations or monovalent metal cations achieved.

Limiting the quantity of monovalent ion present in the depilatory composition may prevent skin irritation but also limits the quantity of thioglycolate salt that may be present in a formula if monovalent ion containing thioglycolate salts or bases are used. Accordingly, in an advantageous embodiment, the depilatory composition comprises a divalent cation" preferably a divalent metal cation, and preferably wherein the thioglycolate salt, the buffering base (if present) or both comprises a divalent cation, or more preferably a divalent metal cation in order to enable the inclusion of additional depilatory active. In another preferred embodiment, the thioglycolate salt comprises a divalent metal cation. Applicants have established that thioglycolate salts comprising monovalent metal cations, such as potassium thioglycolate, are effective at removing hair from the skin, even at low doses, but may expose the skin tissue to harsh chemical conditions, resulting in irritation. On the other hand, thioglycolate salts comprising divalent metal cations, such as calcium thioglycolate, are relatively non-irritating to the skin.

In a depilatory composition comprising a mixture of monovalent and divalent ions, controlling the ratio of divalent ions to monovalent ions may also improve the safety characteristics of the depilatory articles of the present invention. Increasing the concentration of divalent ions relative to the concentration of monovalent ions increases the likelihood that any particular depilatory active species is associated with a divalent ion, rather than the more irritating monovalent ions. On the other hand, increasing the concentration of monovalent ions increases the effectiveness of the depilatory composition. Accordingly, in an alternative embodiment the ratio of the concentration of divalent ions to the concentration of monovalent ions present in the depilatory composition is advantageously in the range of from 400:1 to 0.02:1, preferably from 200:1 to 0.1:1, more preferably 60:1 to 0.3:1, even more preferably from 20:1 to 0.5:1, and even more preferably still from 15:1 1 to 1:1.

The pH of the depilatory composition may advantageously be in the range of from 6 to 13.8, preferably from greater than 7 to 13, more preferably from 9 to 12.9, even more preferably from 10 to 12.8, even more preferably still from 12 to 12.7 and yet more preferably from 12.3 to 12.6 to improve the efficacy of the active ingredient. The depilatory composition may, in a preferred embodiment, comprise at least one base to control the pH. Preferably, the depilatory composition comprises potassium hydroxide; sodium hydroxide; lithium hydroxide; calcium hydroxide; barium hydroxide; caesium hydroxide; sodium hydroxide; ammonium hydroxide; strontium hydroxide; rubidium hydroxide; magnesium hydroxide; zinc hydroxide; sodium carbonate; pyridine; ammonia; alkanolamides (including monoethanolamine, diethanolamine, triethanolamine), phosphates (including tetrasodium phosphate), arginine or mixtures thereof. More preferably, the depilatory composition comprises at least one buffering base, even more preferably the depilatory composition comprises calcium hydroxide, magnesium hydroxide; barium hydroxide; strontium hydroxide; zinc hydroxide; arginine or mixtures thereof. Still more preferably the depilatory composition comprises calcium hydroxide; magnesium hydroxide, zinc hydroxide, sodium hydroxide, potassium hydroxide or mixtures thereof. Even more preferably still, the depilatory composition comprises calcium hydroxide.

In a preferred embodiment, the base is present at a concentration of from 0.1% to 10.0%, more preferably from 0.5% to 8.0% and even more preferably from 1.0% to 5.0%, by weight of the depilatory composition.

In another preferred embodiment, the depilatory composition comprises at least one silicate or silica, advantageously at least one water-soluble or colloid-forming silicate or silica, in order to enhance depilatory effectiveness.

The depilatory composition may optionally comprise a thickening agent. A representative but not exhaustive list can be found in "The Encyclopaedia of Polymers and Thickeners for Cosmetics" compiled and edited by Robert Y. Lochhead, PhD and William R. Fron, Department of Polymer Science, University of Southern Mississippi. Exemplary classes of thickening agents include gums, carbomers, polymers and copolymers of acrylic acid, associated thickeners, layered silicates/clays and natural polymers (including polysaccharides). One or more thickening agents may be included in the depilatory composition. It may be desirable to utilize gel network structures or oil-in-water emulsions to thicken the depilatory compositions. Suitable materials for preparing the gel network structures or oil-in-water emulsions are well represented in the art and include fatty materials such as fatty alcohols (for example cetyl alcohol and stearyl alcohol) alone or used in conjunction with non-polar oils such as paraffin or mineral oils. An appropriate emulsifier may also be used to form and stabilize the bilayer structure characteristic of gel network structures or to form and stabilize an oil-in-water emulsion. The thickening agent may be present at a level of from about 0.01% to about 20%, preferably from about 0.1% to about 10%, more preferably from about 0.3% to about 5%, and even more preferably from about 0.5% to about 4%, by weight of the depilatory composition.

Advantageously, the thickening agent comprises carrageenan. The carrageenan is preferably present in an amount of from 0.1% to 10%, more preferably from 0.5% to 8%, even more preferably from 1% to 5% and even more preferably still from 2% to 4% by weight of the depilatory composition. The carrageenan may be iota, kappa or lambda carrageenan, and in a preferred embodiment is iota carrageenan. Without wishing to be bound by theory, the applicants believe that a depilatory composition comprising carrageenan has both an affinity to the surface of the skin, providing an effect analogous to a frictional resistance opposing spreading of the composition and cohesive forces that further prevent spreading and additionally prevent rupturing of the composition.

Preferably, the depilatory composition comprises at least one water-soluble or colloid-forming silicate selected from lithium silicates; sodium silicates (including disodium metasilicate pentahydrate and disodium metasilicate nanohydrate); potassium silicates; calcium silicates, ammonium silicates; manganese silicates; imidazolium silicates, synthetic and natural silicates (clays) or mixtures thereof. More preferably, the depilatory composition comprises at least one water-soluble or colloid-forming silicate selected from synthetic clays; sodium silicates, potassium silicates, or mixtures thereof and even more preferably the depilatory composition comprises a sodium silicate or mixtures of sodium silicates.

Alternatively, the depilatory composition comprises a form of silica that is colloid-forming, (such as amorphous microporous silica), forms sol or gel systems, (such as silica gels and nano-colloidal silicas), or is mesostructured. Surface modification of silica may be advantageous to promote the formation of stable colloid systems.

Suitable synthetic and natural silicates (clays) are available commercially as: Laponite® RDS; XLS and S etc. (available from RockWood Additives Limited); Wyoming Bentonite; Californian Hectorite; Jadeite; Enstaite and Rhodonite; Benonate® EW (available from Rheox Inc.); Bentolite® (available from Southern Clay Products Inc.) Optigel® (available from Süd Chemie Rheologicals)

The silicate or silica is preferably present in the depilatory composition in an amount per unit area of the coated region of from 2.05 × 10⁻⁸ mol/cm² to 1.23 × 10⁻⁴ mol/cm², preferably from 1.64 × 10⁻⁷ mol/cm² to 3.69 × 10⁻⁵ mol/cm² and more preferably from 4.92 × 10⁻⁷ mol/cm² to 8.20 × 10⁻⁶ mol/cm². Within the preferred ranges, the effectiveness of the depilatory composition is further increased while irritation is maintained within an acceptable level. Without wishing to be bound by theory, applicants believe that an amount of silicate or silica is required in order to enhance the dissociation of the thioglycolate salt sufficiently for the increase in efficacy to be clearly apparent to the user, but that excessive dosage of silicate or silica may lead to over-dissociation of the thioglycolate salt resulting in increased skin irritation. Alternatively, the silicate or silica may be present in the depilatory composition in an amount of from 0.01% to 5%, preferably 0.1% to 4%, more preferably 0.2% to 3% and even more preferably from 0.5% to 2% by weight of the depilatory composition.

The depilatory composition may also include other skin care ingredients such as conditioning agents selected from the group consisting of humectants, moisturizers, or skin conditioners (including mineral oil; almond oil; chamomile oil; jojoba oil; avocado oil; shea butter, niacinamide and glycerine); skin rejuvenation compositions (for example targeted for fine lines, wrinkles and uneven skin tone including retinoids), cosmetic compositions; anti-inflammatory agents (including corticosteroids); anti-oxidants (including flavonoids) radical scavengers; sunscreen agents; skin cooling or warming agents and the like. The depilatory composition may comprise one or more skin care ingredients present in an amount of from about 0.001% to about 10%, more preferably from about 0.01% to about 7%, and even more preferably from about 0.025% to about 5%, by weight of the depilatory composition.

An accelerant may be employed in the depilatory composition. This optional component accelerates the rate of depilatory action of the depilatory agent. Suitable accelerants include, but are not limited to, urea; thiourea; dimethyl isosorbide; arginine salts; ethoxydiglycol; propylene glycol and methylpropyldiol. The accelerant may be present in a concentration range of from 0.5% to 10%, more preferably from 2% to 8% and even more preferably from 2% to 5% by weight of the depilatory composition.

The depilatory composition may further comprise components known, conventionally used, or otherwise effective for use in hair removal compositions particularly dyes; pigments (including ultra marines and talc); anionic, cationic, non-ionic and/or amphoteric or zwitterionic surfactants, polymers (including hydrophobically modified polymers); dispersing agents; solvents; lubricants; fragrances; preservatives; chelants, proteins and derivatives thereof, plant materials (e.g. aloe, chamomile and henna extracts); silicones (volatile or non-volatile, modified or non-modified); film-forming agents; film forming promoters and mixtures thereof.

Depilatory articles of the present invention may take any form suitable for applying to keratinous tissue. The size and shape of the depilatory article may take any form suitable for application to the body area from which hair is to be removed. The depilatory article will preferably relate to the body area or zone from which hair is to be removed, especially the face (including the jaw, chin and upper lip regions of the face), underarm and bikini areas. Preferably, the depilatory article takes the form of a mask (configured for the face) or a strip/patch (configured for general use). In another preferred embodiment, the substrate of the depilatory article is substantially planar.

In a preferred embodiment, the depilatory articles of the present invention are packaged to prevent water loss and/or oxygen permeation. Alternatively, the depilatory articles of the present invention are packaged in water impermeable packaging. Examples of suitable packaging materials include films of EVOH; PP; PE; Nylon; foil laminates (including metalized PET; BOPP and PE), mixtures thereof, laminates thereof or multi-laminates thereof. More preferably, the packaging comprises an inert gas and even more preferably the inert gas comprises at least one of nitrogen, argon or carbon dioxide. Alternatively, the packaging comprises a partial vacuum.

A second aspect being a method of removing hair from the skin is also provided by the present invention, comprising the steps of:
(a) applying a depilatory article according to the present invention to the surface of the skin, preferably mammalian and more preferably human skin,
(b) leaving said depilatory article in contact with the skin for a period of at least 1 minute, preferably 2 to 10 minutes, more preferably 2 to 8 minutes,
(c) removing said depilatory article from the surface of the skin, and
(d) preferably rubbing, scraping, rinsing or wiping the surface of the skin in the area to which the depilatory article was applied.

Advantageously, the method of removing hair from the skin further comprises the step of tensioning the coated region of the depilatory article prior to applying it to the skin.

The same means used to apply tension to the coated region may be used to ensure that the depilatory article is applied to the surface of the body such that the coated region is applied under tension to the unwanted hair in order to maintain the improved handling characteristics described above. In a preferred embodiment, the tension is kept substantially constant during application of the depilatory article. The flexible nature of the substrate allows the substrate to conform to the surface of the body to offer improved contact between the depilatory composition and the unwanted hair. In a preferred embodiment, the tension may be at least partially, more preferably substantially completely released from the coated region after applying the depilatory article to the skin in order to improve the conformability of the depilatory article.

A third aspect being a depilatory kit is also provided by the present invention, which comprises at least one depilatory article of the present invention, packaging for said depilatory article(s), and optionally at least one of a third component selected from:
a) a pre-treatment skin care composition which may comprise ingredients to promote skin conditioning (e.g. emollients), hair hydration or provide a skin barrier (e.g. hydrophobic materials) and intended for use prior to applying the depilatory article.
b) a post-treatment skin care composition which may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like as described herein above. The complementary post treatment skin care compositions may be leave-on or rinse-off compositions. The skin care compositions may also be designed to immediately follow application of the hair removal products. For example, a finishing composition may be applied to the same skin area to combat lingering odour and irritation caused by residual depilatory agent. The finishing composition may comprise a metal oxide (e.g., zinc oxide, aluminum oxide, and magnesium oxide) that is capable of complexing with any remaining depilatory agent remaining on the targeted skin area to reduce continued odour and subsequent skin irritation.
c) a tool to assist in the removal of hair and/or depilatory composition from the skin.
d) indications (which may include instructions and/or illustrations) relating to at least one aspect of usage of the depilatory article or another component of the kit.

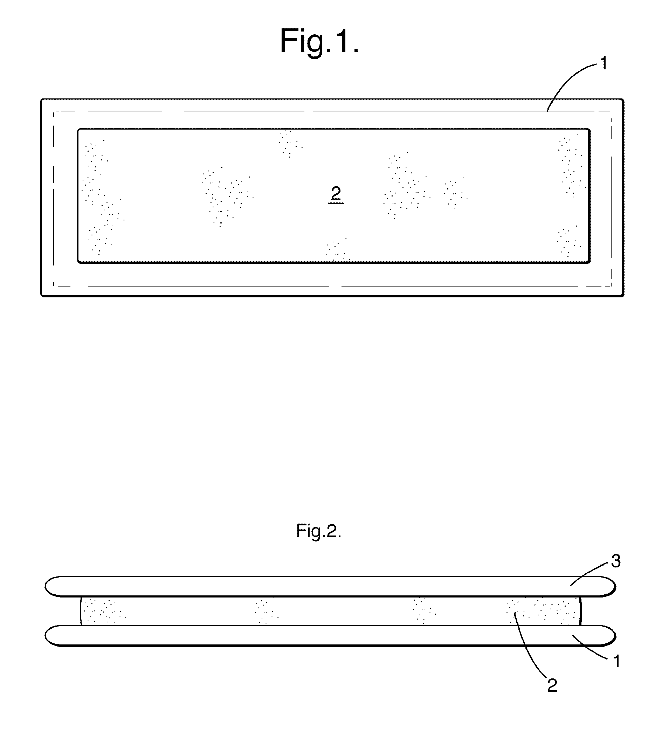

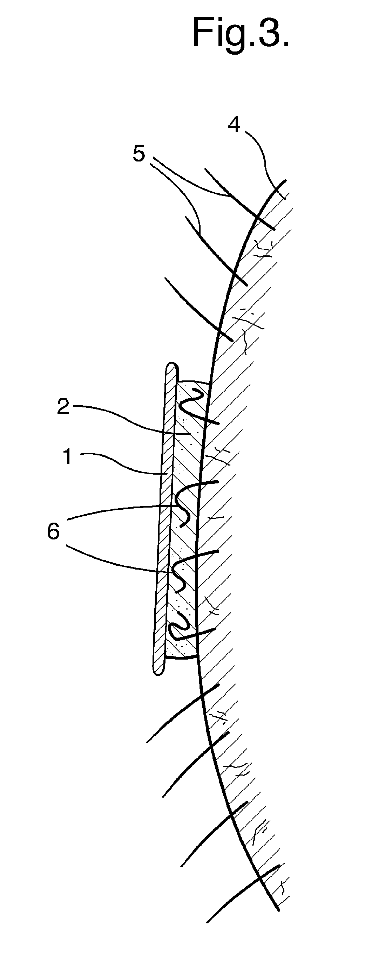

Reference is made to the figures, which disclose a non-limiting embodiment of the invention. Fig.1 depicts a plan view of a depilatory article of the present invention, comprising a substrate (1) and a depilatory composition (2). Fig. 2 depicts a side view of a depilatory article of the present invention, further comprising a protective release layer (3). Fig.3 depicts a side view of a depilatory article of the present invention in use, i.e. applied to keratinous tissue which comprises the skin (4), hair strands (5) outside the depilatory composition(2) and hair strands (6) within the depilatory composition(2).

### Example

The following example further describes and demonstrates one embodiment within the scope of the present invention. The example is given solely for the purpose of illustration and is not to be construed as a limitation of the present invention, as many variations thereof are possible.

### Preparation of the polymeric composition

A polymeric composition is prepared by melting 36% w/w of a ethylene vinyl acetate copolymer (Escorene UL04533EH2 available from Exxon Mobil) and 24% w/w of dibutyl sebacate (available from Sigma-Aldric Co.) mixed at a temperature above their melting point, i.e. at 60-70°C until a homogenous mixture is obtained. The mixture is cooled down to approximately 50°C before adding the volatile material (perfume oil) under stirring conditions so as to obtain a homogenous polymeric composition. The resulting polymeric composition is then cooled down to room temperature.

The polymeric composition is then granulated using an Ekato-Unimix LM3 Homogenizer system to the required particle size to produce the sold entrapping particles. The solid entrapping particles may be prepared as a slurry by the dispersion in water and a thickener (such as Carbopol EDT 2050 neutralized with triethanolamine to pH 4.5 to 5)

Comparative and Inventive depilatory compositions may be exemplified as follows:

| Formulation Ingredients | Comparative example | Inventive example |
|---|---|---|
| | % w/w | % w/w |
| DI water | 86.55 | 85.76 |
| Carrageenan CI-123¹ | 2.60 | 2.60 |
| Sodium silicate solution (42% w/w in water)² | 1.60 | 1.60 |
| Calcium hydroxide³ | 3.25 | 3.25 |
| Calcium thioglycolate trihydrate⁴ | 5.50 | 5.50 |
| Perfume oil | 0.50 | - |
| Solid entrapping particles (see above)⁵ | - | 1.29 |

| | | |
|---|---|---|
| 1 Carrageenan CI-123 available from CPKelco. 2 Sodium silicate solution (42% w/w in water) under the tradename Cognis 60 available from Cognis. 3 Calcium Hydroxide Reag. Ph. Eur. puriss. p.a. available from Sigma-Aldrich Co. 4 Calcium Thioglycolate Trihydrate 99.8% available from BRUNO BOCK Chemische Fabrik GmbH & Co. 5 Solid entrapping particles contained 12% w/w of the same perfume oil used in the comparative example (such that the total perfume oil in both examples is 0.5% w/w). | | |

A 400 ml speed mixer plastic pot was sanitized and DI water weighed in. The calcium hydroxide was added with mixing, followed by the slow addition of the perfume oil. The Carrageenan was then slowly added to the batch with mixing to ensure hydration, adjusting mixing speeds and time as required. The calcium thioglycolate was then added with mixing, again adjusting mixing speeds and time as required. The batch was then milled using an IKA T50 (5,200) rpm for 2 minutes. The pH was measured and the batch stored in glass pots with no head space. The above procedure was repeated for the inventive example by adding the solid entrapping particles instead of the perfume oil.

The above comparative depilatory composition was disposed to a thickness of 0.3 mm, width of 3.0 cm and length of 3.5 cm onto a 23 micron thick HDPE 85%/LDPE 15% film (4.6 cm in length and 3.2 cm in width) using a stencil and wiper blade, such that the area covered by the depilatory composition was centered along the width of the film and 1 mm away from the perimeter edge of one end of the film's length, thus providing a 1 mm overhang of substrate at the edges of the depilatory composition along the length and at one edge across it width. The other edges across the width of depilatory composition therefore had an overhang of 10 mm. An 89 micron thick release layer (SBOPP available from 3M Company under the tradename 3M Scotchpak^{™} 9741) was then carefully positioned directly on the depilatory composition such that the edges were above the edges of the substrate. The strip was sealed in a 7 micron foil laminate pouch under N₂ and then stored at 40°C for 3 days. The procedure was repeated for the inventive depilatory composition.

The depilatory compositions for both the comparative and inventive examples were then evaluated by a qualified perfumer after removing the depilatory composition from the substrates with a spatula. While the inventive example remained pleasantly fragrant, the comparative example held no detectable fragrance. A standard Gas Chromatography - Mass Spectrometry experiment also confirmed that 95% of the perfume oil had been lost from the depilatory composition of the comparative example.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A depilatory article comprising:
i) a substrate; and
ii) a depilatory composition disposed on said substrate,
wherein the depilatory composition comprises solid entrapping particles, said particles comprising:
i) a polymer; and
ii) a volatile material or mixture of volatile materials.

2. A depilatory article according to claim 1 wherein the particles have an average particle size from 5 to 200 micrometers, preferably from 30 to 100 micrometers, more preferably from 30 to 50 micrometers.

3. A depilatory article according to either preceding claim wherein the volatile material or mixture of volatile materials is a perfume, preferably selected from a perfume comprising an aldehyde, a ketone, an alcohol, a terpene, an ester or a mixture thereof.

4. A depilatory article according to any preceding claim wherein said depilatory composition comprises from 0.01% to 10%, preferably from 0.1% to 5%, more preferably from 0.2% to 3% even more preferably from 0.5% to 2% and even more still preferably from 0.7% to 1.5%solid entrapping particles by weight of the depilatory composition.

5. A depilatory article according to any preceding claim wherein said polymer has a melting point from 45°C to 80°C, preferably from 45°C to 55°C.

6. A depilatory article according to any preceding claim, wherein the polymer is a copolymer obtainable by copolymerization of monomers of ethylene with at least one class of co-monomers comprising at least one heteroatom, preferably wherein said co-monomer comprises a carbonyl group, more preferably said co-monomer comprises an ester group, even more preferably said co-monomer is selected from a vinyl ester, an acrylic ester, a methacrylic ester and mixtures thereof and still more preferably said co-monomer is a vinyl acetate ester.

7. A depilatory article according to any preceding claim, wherein the solid entrapping particles comprise from 10% to 98%, preferably from 15% to 80%, more preferably from 18% to 50%, and even more preferably from 20% to 40%, volatile material by weight of particle

8. A depilatory article according to any preceding claim, wherein the particles further comprise a plasticizer, preferably a plasticizer comprising at least one heteroatom, more preferably a plasticizer comprising a carbonyl group, even more preferably a plasticizer comprising an ester group, even more preferably still a plasticizer selected from adipic acid esters, benzoic acid esters, citric acid esters, phosphoric acid esters, phthalic acid esters, rosin esters, sebacic acid esters, sucrose esters, tartaric acid esters or mixture thereof and yet more preferably a plasticizer selected from adipic acid esters, citric acid esters, rosin esters, sebacic acid esters or mixtures thereof.

9. A depilatory article according to claim 8, wherein the solid entrapping particles comprise, by weight of particle, from 5% to 75% copolymer, from 10% to 50% plasticizer and from 10 to 85% volatile material.

10. A depilatory article according to any preceding claim, wherein the depilatory composition is in the form of an aqueous gel, preferably wherein the depilatory composition comprises water in an amount of at least 40%, preferably from 50% to 98%, more preferably from 60% to 95% and even more preferably from 70% to 90%, by weight of the depilatory composition.

11. A depilatory article according to any preceding claim, wherein a protective release layer (3) is removably attached to the depilatory composition of the depilatory article.

12. A depilatory article according to any preceding claim, wherein the substrate and/or release layer is water impermeable.

13. A depilatory article according to any preceding claim, wherein said depilatory composition is disposed on said substrate in an amount per unit area of the coated region of from 0.3 g/cm² to 0.001 g/cm², preferably from 0.015 g/cm² to 0.003 g/cm², more preferably 0.08 g/cm² to 0.005 g/cm² and even more preferably from 0.05 g/cm² to 0.005 g/cm².

14. A method of removing hair from the skin, comprising the steps of:
(a) applying a depilatory article according to any preceding claim to a surface of skin, preferably human skin,
(b) leaving said depilatory article in contact with the skin for a period of greater than 1 minute, preferably 2 to 10 minutes, more preferably 2 to 8 minutes
(c) removing said depilatory article from the surface of the skin, and
(d) preferably rubbing, scraping, rinsing or wiping the surface of the skin in the area to which the depilatory article was applied.

15. A depilatory kit, comprising:
(a) a depilatory article according to any of claims 1-13,
(b) optionally, at least one of a pre-treatment skin care composition, a post-treatment skin care composition and/or a tool to assist removal of hair and/or depilatory composition after use, and
(c) packaging for said depilatory kit.
